# EUROPÄISCHE PATENTSCHRIFT

(11) **EP 3 335 526 B1**
(45) Veröffentlichungstag und Bekanntmachung des Hinweises auf die Patenterteilung: **08.12.2021**
(21) Anmeldenummer: 16751279.7
(22) Anmeldetag: 08.08.2016
(51) Int. Cl.: F21S 8/00, F21V 5/00, F21V 23/04, F21Y 115/10, F21Y 113/10, F21W 131/205

(54) **OPERATIONSLEUCHTE MIT VERÄNDERBARER LICHTFELDGEOMETRIE**
SURGICAL LIGHT HAVING A VARIABLE LIGHT FIELD GEOMETRY
SCIALYTIQUE À GÉOMÉTRIE MODIFIABLE DU CHAMP LUMINEUX

(30) Priorität: 13.08.2015 DE 102015113337
(43) Veröffentlichungstag der Anmeldung: 20.06.2018
(73) Patentinhaber: Karl Leibinger Medizintechnik GmbH & Co. KG, 78570 Mühlheim/Donau (DE)
(72) Erfinder: STRÖLIN, Joachim, 78604 Rietheim (DE)
(74) Vertreter: Winter, Brandl - Partnerschaft mbB
(86) Internationale Anmeldenummer: PCT/EP2016/068892
(87) Internationale Veröffentlichungsnummer: WO 2017/025512

(56) Entgegenhaltungen:
- EP-A1- 1 568 935
- EP-A1- 1 722 157
- EP-A1- 2 136 126
- EP-A1- 2 136 128
- DE-U1-202007 015 823

## Beschreibung

Die Erfindung betrifft eine Operationsleuchte zum Ausleuchten eines Wundfeldes nach dem Oberbegriff des Anspruchs 1, mit einer Vielzahl einer Leuchtengruppe zugeordneten Leuchten, welche Leuchten jeweils (in einem eingeschalteten / bestromten Zustand) ein sich entlang einer Längsachse erstreckendes Lichtstrahlbündel erzeugen sowie derart ausgerichtet und zueinander angeordnet sind, dass sich die Längsachsen ihrer Lichtstrahlbündel in einer gemeinsamen Fokusebene schneiden. Insbesondere sind mehrere erste Leuchten einer ersten Leuchtengruppe zugeordnet und mehrere zweite Leuchten sind einer zweiten Leuchtengruppe zugeordnet, wobei sich die Längsachsen der Lichtstrahlbündel der ersten Leuchten in einer ersten gemeinsamen Fokusebene schneiden und die Längsachsen der Lichtstrahlbündel der zweiten Leuchten in einer, beabstandet zu der ersten Fokusebene angeordneten, zweiten gemeinsamen Fokusebene schneiden.

Gattungsgemäßer Stand der Technik ist etwa aus der EP 2 136 128 A1 bekannt, die eine Operationsleuchte umfassend einen Leuchtenkörper mit einer Mittelachse mit mindestens zwei Leuchtmitteln mit gebündelten Lichtstrahlen umfasst. Eine Achse einzelner Lichtstrahlbündel ist dabei auf jeweils einen Punkt auf einer Mittelachse gerichtet, wobei ein Abstand der jeweiligen Punkte zum Leuchtenkörper in Richtung der Mittelachse unterschiedlich ist. Weiterer Stand der Technik ist aus der EP 2 136 126 A1 bekannt.

Hierbei hat es sich herausgestellt, dass die aus dem Stand der Technik bekannten Operationsleuchten bei relativ komplexen Operationsbereichen / Wundfeldern am Körper, insbesondere in Bezug auf die Ausleuchtung noch relativ schwierig oder gar nicht an die individuellen Operationsbereiche anpassbar sind. Vor allem eine Anpassung der Ausleuchtung an sich während der Operation ändernde Wundfelder ist hierdurch schwierig umzusetzen. Da bei sich formlich ändernden Wundfeldern alle Leuchten der jeweiligen Gruppe bei den bekannten Ausführungen erhellt oder abgedunkelt werden müssen, ist es von Nachteil, dass daraufhin in der Ausleuchtebene der gesamte Bereich gleichmäßig erhellt oder abgedunkelt wird. Dies ist jedoch insbesondere dann unerwünscht, wenn das Wundfeld eine von einer kreisrunden Ausformung abweichende Form aufweist und sich etwa länglich erstreckt. Denn dann werden auch die seitlich an das längliche Wundfeld angrenzenden Hautbereiche stark überhellt, wodurch es gar zum Blenden des Operateurs kommen kann, oder das Wundfeld nicht ausreichend hell beleuchtet, wodurch das Wundfeld wiederum schwierig zu erkennen ist.

Es ist daher die Aufgabe der vorliegenden Erfindung, diese aus dem Stand der Technik bekannten Nachteile zu beheben und eine Operationsleuchte zur Verfügung zu stellen, die unabhängig von der Form des jeweiligen Wundfeldes des operierten Körpers eine gleichmäßige Ausleuchtung des Wundfeldes ermöglichen soll.

Dies wird erfindungsgemäß dadurch gelöst, dass die Leuchten der Leuchtengruppe unabhängig voneinander bestrombar (d.h. elektrisch versorgbar / betätigbar) sind, sodass eine durch die Leuchten der Leuchtengruppe (d.h. durch die Lichtstrahlbündel der Leuchten) erzeugte Lichtfeldgeometrie in einer beabstandet zur Fokusebene angeordneten Ausleuchtebene einstellbar ist. Zudem ist eine (zentrale) Steuereinheit in der Operationsleuchte vorhanden, die elektrisch mit jeder einer Leuchtengruppe zugeordneten Leuchte (d.h. mit jeder (ersten) Leuchte der ersten Leuchtengruppe und mit jeder (zweiten) Leuchte der zweiten Leuchtengruppe) elektrisch verbunden ist.

Dies ermöglicht erfindungsgemäß eine individuelle geometrische Einstellung des erzeugten Gesamtlichtfeldes, das durch die Lichtstrahlbündel der einzelnen Leuchten (auch als Einzelleuchten bezeichnet) der Leuchtengruppe erzeugt wird. Hierdurch kann das resultierende Gesamtlichtfeld nicht nur kreisrund, sondern auch in weiteren Formen, etwa einer ovalen oder länglichen Form, beliebig eingestellt und "verdreht" werden. Ein solches Gesamtlichtfeld kann durch die jeweilige Betätigung einzelner Leuchten der Leuchtengruppe gar um 360° "verdreht" werden, wodurch das Lichtfeld dem Wundfeld optimal angepasst werden kann. Zudem lassen sich die Lichtfeldgeometrien durch eine besonders direkte Anbindung umschalten. Auch wird eine verlässliche Ausleuchtung in mehreren Ebenen umgesetzt. Die erste Fokusebene bildet dabei bspw. eine (zweite) Ausleuchtebene der zweiten Leuchten aus, während die zweite Fokusebene eine (erste) Ausleuchtebene der ersten Leuchten ausbildet.

Weitere vorteilhafte Ausführungsformen sind in den Unteransprüchen beansprucht und nachfolgend näher erläutert.

In diesem Zusammenhang ist vorteilhaft, wenn sowohl die (ersten) Leuchten der ersten Leuchtengruppe als auch die (zweiten) Leuchten der zweiten Leuchtengruppe unabhängig voneinander bestrombar / elektrisch betätigbar sind, sodass durch die Leuchten jeder (erste oder zweite) Leuchtengruppe die auf der jeweiligen Ausleuchtebene (erste oder zweite Ausleuchtebene) erzeugte Lichtfeldgeometrie einstellbar ist. Daraus resultiert, dass die Leuchtfeldgeometrien der Leuchtengruppen noch individueller angepasst werden können.

Im Weiteren ist es vorteilhaft, wenn die (ersten) Leuchten der ersten Leuchtengruppe und/oder die (zweiten) Leuchten der zweiten Leuchtengruppe in einem gemeinsamen Leuchtenaufnahmekörper angeordnet sind. Dadurch ist die Relativposition der einzelnen Leuchten der unterschiedlichen Leuchtengruppen zueinander festgelegt.

Zweckmäßig ist es auch, wenn die die einer Leuchtengruppe zugeordneten Leuchten (d.h. die (ersten) Leuchten der ersten Leuchtengruppe und/oder die (zweiten) Leuchten der zweiten Leuchtengruppe) jeweils aus einem, eine LED umfassenden (vorzugsweise einzeln ausgeformten) Leuchtenmodul ausgebildet sind. Dadurch ist das jeweilige Gesamt-/Lichtfeld / die jeweilige Lichtfeldgeometrie besonders einfach einstellbar, wobei jede Leuchte nur einen Lichtpunkt (d.h. ein im Wesentlichen kreisförmiges / ovales Teillichtfeld) ausbildet, der ein bzw. ausschaltbar ist. Durch Aktivierung mehrerer entlang einer Längsachse angeordneter Leuchten (der gleichen oder eine unterschiedlichen Leuchtengruppe) ergibt sich dann eine Kette nebeneinander angeordneter oder sich teilweise überlappender Lichtpunkte. Dadurch ist eine besonders individuelle Einstellung umsetzbar.

Weiterhin ist es auch vorteilhaft, wenn den einer Leuchtengruppe zugeordneten Leuchten (d.h. den (ersten) Leuchten der ersten Leuchtengruppe und/oder den (zweiten) Leuchten der zweiten Leuchtengruppe) jeweils eine eigene (vorzugsweise verstellbare) Linse / Linsenoptik zugeordnet ist. Die Linse ist dabei ebenfalls Bestandteil des Leuchtenmoduls der Leuchte. Dadurch ist eine besonders einfach Ansteuerung der einzelnen Leuchten möglich, wobei die Brennweite / der Brennpunkt der Leuchten bei Bedarf individuell eingestellt werden kann.

In diesem Zusammenhang ist es auch zweckmäßig, die einer Leuchtengruppe zugeordneten Leuchten (d.h. die (ersten) Leuchten der ersten Leuchtengruppe und/oder die (zweiten) Leuchten der zweiten Leuchtengruppe) unabhängig voneinander in ihrer Helligkeit / Beleuchtungsstärke einstellbar sind. Dadurch ist eine noch individuellere Einstellung des resultierenden Gesamtlichtfeldes umgesetzt.

Von Vorteil ist es zudem, wenn sich zumindest einige der einer Leuchtengruppe zugeordneten Leuchten (d.h. einige der (ersten) Leuchten der ersten Leuchtengruppe und/oder einige der (zweiten) Leuchten der zweiten Leuchtengruppe) voneinander durch ihre Lichtfarbe unterscheiden. Dadurch lässt sich durch die individuelle Ansteuerung / Bestromung der Leuchten auch das resultierend Gesamtlichtfeld farblich verstellen.

Im Weiteren ist es auch vorteilhaft, wenn die einer Leuchtengruppe zugeordneten Leuchten (d.h. die (ersten) Leuchten der ersten Leuchtengruppe und/oder die (zweiten) Leuchten der zweiten Leuchtengruppe) ringförmig nebeneinander angeordnet sind. Dadurch sind alle Leuchten einfach um die Mittelachse der Operationsleuchte herum, in einem gleichen Winkel zu der Mittelachse geneigt anzuordnen. Der Aufbau wird dadurch weiter vereinfacht.

In diesem Zusammenhang ist es besonders zweckmäßig, wenn die der ersten Leuchtengruppe zugeordneten ersten Leuchten entlang einer ersten ringförmigen Umfangslinie (in Bezug auf eine Mittelachse der Operationsleuchte) verteilt angeordnet sind sowie die der zweiten Leuchtengruppe zugeordneten zweiten Leuchten entlang einer zweiten ringförmigen Umfangslinie (in Bezug auf eine Mittelachse der Operationsleuchte) verteilt angeordnet sind. Dadurch lässt sich in möglichst vielen, unterschiedlichen Verdrehpositionen das Gesamtlichtfeld ausbilden.

Ist die erste Umfangslinie dabei innerhalb der zweiten Umfangslinie angeordnet, ist der Aufbau der Operationsleuchte weiter vereinfacht ausgeführt.

Im Weiteren ist es vorteilhaft, wenn eine Bedienungseinrichtung vorhanden ist, mittels der die Lichtfeldgeometrie einstellbar ist. Dadurch ist eine individuelle Einstellung durch den Operateur ermöglicht.

Wenn die Bedienungseinrichtung weiterhin eine Spracherkennungseinheit aufweist, ist das Gesamtlichtfeld / die Lichtfeldgeometrie der Operationsleuchte auch individuell unmittelbar, ohne Berühren der Bedienungseinrichtung einstellbar. Dadurch wird die Hygiene weiter verbessert.

In diesem Zusammenhang ist es weiterhin auch von Vorteil, wenn die Bedienungseinrichtung mittels einer drahtgebundenen oder drahtlosen Datenverbindung mit der Steuereinheit verbunden ist. Somit wird die Bedienung weiter vereinfacht.

Die Erfindung wird nun nachfolgend anhand von Figuren näher erläutert.

Es zeigen:
- Fig. 1: eine schematische Seitendarstellung einer erfindungsgemäßen Operationsleuchte nach einem vorteilhaften Ausführungsbeispiel, wobei die Lichtstrahlbündel zweier erster Leuchten, die einer ersten Leuchtengruppe zugeordnet sind, zu erkennen sind und hierbei insbesondere deren gemeinsamer Schnittpunkt in der ersten Fokusebene verdeutlich ist, während die einer zweiten Leuchtengruppe zugeordneten (zweiten) Leuchten allesamt abgeschaltet sind,
- Fig. 2: eine schematische Seitendarstellung der Operationsleuchte gemäß Fig. 1, wobei nun die ersten Leuchten der ersten Leuchtengruppe allesamt abgeschaltet sind, stattdessen zwei zweite Leuchten der zweiten Leuchtengruppe eingeschaltet sind, und wiederum der gemeinsame Schnittpunkt derer Lichtstrahlbündel in einer, zu der ersten Fokusebene beabstandeten, zweiten Fokusebene erkennbar ist,
- Fig. 3: eine schematische Seitendarstellung der Operationsleuchte gemäß den Fign. 1 und 2, wobei nun sowohl zwei (erste) Leuchten der ersten Leuchtengruppe als auch zwei (zweite) Leuchten der zweiten Leuchtengruppe eingeschaltet sind und die sich überlagernden Teillichtfelder derer Lichtstrahlbündel in der ersten Fokusebene gut zu erkennen sind,
- Fig. 4: eine schematische Draufsicht auf eine Ausleuchtebene, in der die Leuchten der ersten und zweiten Leuchtengruppe der Operationsleuchte derart angesteuert sind, dass sich ein resultierendes (Gesamt-)Lichtfeld ergibt, das im Wesentlichen linienförmig verläuft, vorzugsweise entlang einer Horizontalachse,
- Fig. 5: eine Draufsicht auf die Ausleuchtebene gemäß Fig. 4, wobei nun die Leuchten der ersten und zweiten Leuchtengruppe derart angesteuert sind, dass sich ein im Vergleich zur Fig. 4 um 45° "verdrehtes", längliches Gesamtlichtfeld ergibt,
- Fig. 6: eine Draufsicht auf die Ausleuchtebene gemäß Fig. 4, wobei nun die Leuchten der ersten und zweiten Leuchtengruppe derart angesteuert sind, dass sich ein im Wesentlichen H-förmiges Gesamtlichtfeld ergibt,
- Fig. 7: eine Draufsicht auf die Ausleuchtebene gemäß Fig. 4, wobei nun die Leuchten der ersten und zweiten Leuchtengruppe derart angesteuert sind, dass sich ein im Wesentlichen kreuzförmiges Gesamtlichtfeld ergibt, und
- Fig. 8: eine Draufsicht auf die Ausleuchtebene gemäß Fig. 4, wobei nun die Leuchten der ersten und zweiten Leuchtengruppe derart angesteuert sind, dass sich ein im Wesentlichen dreieckförmiges Gesamtlichtfeld ergibt.

Die Figuren sind lediglich schematischer Natur und dienen ausschließlich dem Verständnis der Erfindung. Die gleichen Elemente sind mit denselben Bezugszeichen versehen.

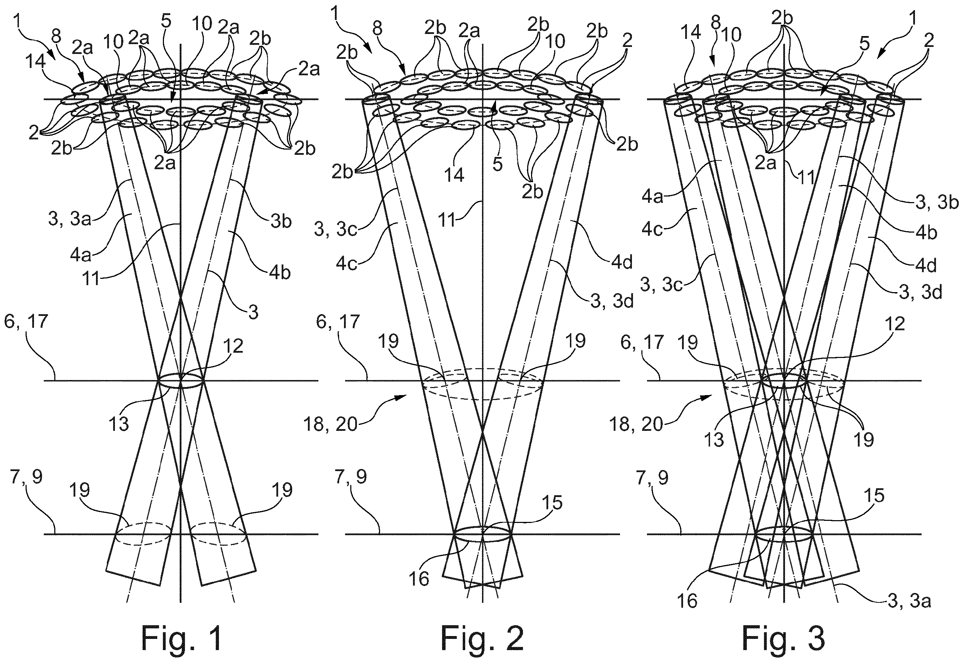

Die erfindungsgemäße Operationsleuchte 1 ist in Fig. 1 zunächst in ihrem schematischen Aufbau besonders gut zu erkennen. Die Operationsleuchte 1 dient hierbei auf übliche Weise dem Ausleuchten / Beleuchten eines auf einem Behandlungstisch befindlichen Objekts, etwa einem Menschen. Die Operationsleuchte 1 ist daher für das Ausleuchten eines Operationsbereiches an diesem Objekt, nämlich eines Wundfeldes ausgelegt.

Die Operationsleuchte 1 weist eine Vielzahl an einzelnen Leuchten 2 auf. Jede Leuchte besteht dabei aus einem einzelnen, d.h. stofflich separat von den übrigen Leuchten 2 ausgeformten Leuchtenmodul. Das Leuchtenmodul umfasst wiederum eine LED-Lampe / -Birne / ein LED-Licht und eine zugehörigen Linse / Linsenoptik. Die Leuchten 2 weisen dabei in ihrem Leuchtenmodul jeweils stets nur eine LED auf. Auch weist jedes Leuchtenmodul dabei entsprechende Reflektoren bzw. Vorrichtungen zum Bündeln des durch die LED imitierten Lichts auf, welches Licht seitens der Linse aus dem Leuchtenmodul in Form eines Lichtstrahlbündels 4 austritt. Somit bildet jede Leuchte 2 in einem eingeschalteten / bestromten Zustand ein sich entlang einer Längsachse 3 erstreckendes Lichtstrahlbündel 4 aus. In anderen Worten ausgedrückt, erzeugt jede Leuchte 2 bzw. jedes Leuchtmodul der Leuchte 2 ein Lichtstrahlbündel 4.

In Fig. 1 sind schematisch zwei erste Leuchten 2a, die einer ersten Leuchtengruppe 5 / Linsengruppe zugeordnet sind, angeschaltet. Die erste Leuchtengruppe 5 besteht in dieser Ausführung nicht nur aus zwei, sondern aus mehr als zwei ersten Leuchten 2a. Insgesamt sind in der ersten Leuchtengruppe 5 zwölf erste Leuchten 2a enthalten. In weiteren Ausführungen beträgt die Anzahl der ersten Leuchten 2a der ersten Leuchtengruppe 5 jedoch auch mehr als zwölf oder weniger als zwölf.

Die ersten Leuchten 2a der ersten Leuchtengruppe 5 sind entlang einer ringförmigen / kreisringförmigen Umfangslinie, die nachfolgend als erste Umfangslinie 10 bezeichnet ist, angeordnet. Die erste Umfangslinie 10 ist dabei zentrisch zu einer gedachten Mittelachse 11 der Operationsleuchte 1 angeordnet. Die Mittelachse 11 der Operationsleuchte 1 bildet dabei im Betrieb jene Mittelachse 11 eines hier der Übersichtlichkeit halber nicht weiter dargestellten Leuchtenaufnahmekörpers der Operationsleuchte 1 aus. Der Leuchtenaufnahmekörper ist jener Körper, an dem die Vielzahl an einzelnen Leuchten 2 aufgenommen / befestigt ist. Folglich sind die Leuchten 2 allesamt an diesem Leuchtenaufnahmekörper befestigt. Im Weiteren ist die Mittelachse 11 auch jene Achse, die sich im Zentrum des Leuchtenaufnahmekörpers der Operationsleuchte 1 befindet und im Wesentlichen entlang eines hier der Übersichtlichkeit halber nicht weiter ausgeführten Handgriffes der Operationsleuchte 1 erstreckt. Insbesondere ist die Mittelachse 11 dabei jene Achse, entlang welcher sich ein zapfenförmiger Griffabschnitt des Handgriffes, der von dem Operateur berührbar ist, erstreckt.

Somit sind die ersten Leuchten 2a der ersten Leuchtengruppe 5 in einer Umfangsrichtung in Bezug auf die Mittelachse 11 nebeneinander, kettenförmig, angeordnet / aneinandergereiht. Alle ersten Leuchten 2a sind dabei derart in einem Winkel relativ zu der Mittelachse 11 ausgerichtet, dass sich deren Längsachsen 3 (die Längsachsen 3 sind jene Achsen, entlang der sich das Lichtstrahlbündel 4 erstreckt) allesamt in einem gemeinsamen Schnittpunkt, hier als erster Schnittpunkt 12 bezeichnet, der in einer gemeinsamen ersten Fokusebene 6 befindlich ist, schneiden. Die Längsachsen 3 der ersten Leuchten 2a weisen daher allesamt einen gleichen Winkel gegenüber der Mittelachse 11 auf. Von dem Leuchtenmodul der Leuchten 2a aus erstreckt sich das jeweilige Lichtstrahlbündel 4 deshalb stets zu der Mittelachse 11 hin, so dass sich der gemeinsamer Fokuspunkt / Schnittpunkt -erster Schnittpunkt 12 - in der ersten Fokusebene 6 ausbildet. Da sich alle ersten Leuchten 2a der ersten Leuchtengruppe 5 in ihrem eingeschalteten Zustand in diesem gemeinsamen, in der ersten Fokusebene 6 befindlichen ersten Schnittpunkt 12 schneiden / überlappen / überdecken bilden diese ein gemeinsames, erstes rundes Fokuslichtfeld 13 aus. Das erste Fokuslichtfeld 13 weißt dabei einen maximalen, ersten Durchmesser von ca. 300 mm auf.

Schematisch sind in Fig. 1 zwei sich in Bezug auf die Mittelachse 11 gegenüberliegende erste Leuchten 2a eingeschaltet, so dass eine der ersten Leuchten 2a ein sich entlang einer ersten Längsachse 3a erstreckendes erstes Lichtstrahlbündel 4a erzeugt und eine, um etwa 180° entlang der ersten Umfangslinie 10 versetzt angeordnete, andere erste Leuchte 2a ein sich entlang einer zweiten Längsachse 3b erstreckendes zweites Lichtstrahlbündel 4b ausbildet. Bis zum ersten Schnittpunkt 12 erstrecken sich die beiden Lichtstrahlbündel 4a, 4b aufeinander zu und von einer der Operationsleuchte 1 abgewandten Seite der ersten Fokusebene 6 aus erstrecken sich diese beiden Lichtstrahlbündel 4a und 4b dann wiederum voneinander weg, stets in einem gleichen Winkel (in Bezug auf den Winkelbetrag) zwischen der jeweiligen Längsachse 3a, 3b zur Mittelachse 11 betrachtet.

In einer beabstandet zur ersten Fokusebene 6 angeordneten ersten Ausleuchtebene 7, auch als Ausleuchtebene schlicht bezeichnet, bilden / leuchten die Lichtstrahlbündel 4a und 4b der beiden ersten Leuchten 2a jeweils ein Teillichtfeld 19 aus.

Wie dann in Zusammenwirkung mit Fig. 2 zu erkennen ist, ist neben der ersten Gruppe / Leuchtengruppe 5 der ersten Leuchten 2a eine zweite Leuchtengruppe 8 / Linsengruppe vorgesehen, die wiederum mehrere der Leuchten 2 aufweist. Die als zweite Leuchten 2b der zweiten Leuchtengruppe 8 bezeichneten Leuchten 2 sind wie die ersten Leuchten 2a aufgebaut und funktionierend. Somit weisen auch die zweiten Leuchten 2b jeweils ein Leuchtenmodul mit nur einer LED und einer dieser LED zugeordneten Linse auf.

Die zweiten Leuchten 2b der zweiten Leuchtengruppe 8 sind in Bezug auf die Mittelachse 11 gesehen radial außerhalb der ersten Leuchten 2a der ersten Leuchtengruppe 5 angeordnet. Auch die zweiten Leuchten 2b sind auf einer Umfangslinie, die nachfolgend als zweite Umfangslinie 14 bezeichnet ist, kreisförmig nebeneinander angeordnet. Somit sind auch die zweiten Leuchten 2b in Umfangsrichtung der Mittelachse 11 angeordnet. Die zweite Umfangslinie 14 weist folglich einen größeren Durchmesser auf als die erste Umfangslinie 10 auf.

Auch sind wiederum in der zweiten Leuchtengruppe 8 nicht nur zwei (zweite) Leuchten 2b, sondern mehr als zwei (zweite) Leuchten 2b eingesetzt. Insgesamt sind achtzehn zweite Leuchten 2b in der zweiten Leuchtengruppe 8 enthalten und kettenartig entlang der kreisrunden zweiten Umfangslinie 14 aneinander gereiht. Jedoch ist in weiteren Ausführungen auch eine andere Anzahl an zweiten Leuchten 2b in der zweiten Leuchtengruppe 8 umgesetzt, wie mehr als achtzehn oder weniger als achtzehn. Zudem sei darauf verwiesen, dass jede der Leuchten 2a und 2b der beiden ersten und zweiten Leuchtengruppen 5 und 8 nicht zwingend eine sich entlang einer kreisförmigen Umfangslinie 10, 14 erstreckende kreisförmige Anordnung aufweisen muss. Auch ist es denkbar, die Leuchtengruppen 5, 8 anders anzuordnen, ohne sich von dem Erfindungsgedanken zu entfernen.

Zurückkommend auf Fig. 2 ist zu erkennen, dass die zweiten Leuchten 2b wiederum allesamt in Richtung der Mittelachse 11 ausgerichtet sind. Alle zweiten Leuchten 2b schließen wiederum mit ihren Längsachsen 3 der Lichtstrahlbündel 4 einen Winkel mit der Mittelachse 11 ein. Die Längsachsen 3 aller zweiten Leuchten 2b weisen hierbei einen gleichen Winkel gegenüber der Mittelachse 11 auf.

Schematisch sind in Fig. 2 wiederum zwei eingeschaltete Leuchten 2b zu erkennen, die sich in Bezug auf die zweite Umfangslinie 14 im Wesentlichen um 180° gegenüber liegen. Eine der beiden zweiten Leuchten 2b erzeugt ein als drittes Lichtstrahlbündel 4c bezeichnetes Lichtstrahlbündel 4, das sich entlang der dritten Längsachse 3c erstreckt. Die andere der beiden zweiten Leuchten 2b erzeugt im eingeschalteten Zustand wiederum ein viertes Lichtstrahlbündel 4d, das sich entlang einer vierten Längsachse 3d erstreckt. Die beiden zweiten Leuchten 2b sind derart aufeinander abgestimmt, dass sich ihre Längsachsen 3c bzw. 3d in einem gemeinsamen Fokuspunkt / Schnittpunkt - nachfolgend als zweiter Schnittpunkt 15 bezeichnet - schneiden. Dieser zweite Schnittpunkt 15 liegt in einer zweiten Fokusebene 9, die gegenüber der ersten Fokusebene 6 beabstandet angeordnet ist. Weiterhin schneiden sich nicht nur die Längsachsen 3c, 3d der beiden in Fig. 2 eingeschalteten zweiten Leuchten 2b, sondern alle in der zweite Leuchtengruppe 8 enthaltenen zweiten Leuchten 2b mit ihren Längsachsen 3 in diesem gemeinsamen zweiten Schnittpunkt 15 in der zweiten Fokusebene 9.

Jede Fokusebene 6 bzw. 9 ist in dieser Ausführung als eine Normalebene relativ zur Mittelachse 11 ausgestaltet. In dieser Ausführungsform bildet die zweite Fokusebene 9 die erste Ausleuchtebene 7 der ersten Leuchtengruppe 5 aus. Die erste Fokusebene 6 bildet wiederum eine (zweite) Ausleuchtebene 17 für die zweite Leuchtengruppe 8 aus, weshalb die beiden Lichtstrahlbündel 4c, 4d der beiden zweiten Leuchten 2b in dieser zweiten Ausleuchtebene 17 wiederum zwei, zueinander beabstandet angeordnete Teillichtfelder 19 ausbilden. Da sich alle zweiten Leuchten 2a in ihrem eingeschalteten Zustand in diesem gemeinsamen zweiten Schnittpunkt 15 schneiden / überlappen / überdecken, bilden diese ein gemeinsames, (zweites) rundes Fokuslichtfeld 16 in der zweiten Fokusebene 9 aus. Das zweite Fokuslichtfeld 16 weißt dabei einen maximalen, zweiten Durchmesser von ca. 150 mm auf. In einer gegenüber der zweiten Fokusebene 9 beabstandeten zweiten Ausleuchtebene 17, die hier der ersten Fokusebene 6 entspricht, bilden die zweiten Leuchten 2b somit wiederum die beabstandet voneinander angeordneten runden Teillichtfelder 19 aus.

Wie weiterhin zu erkennen, ist die erste Fokusebene 6 entlang der Mittelachse 11 betrachtet, näher an der Operationsleuchte 1, d.h. an dem Leuchtenaufnahmekörper der Operationsleuchte 1, angeordnet als die zweite Fokusebene 9. Daher weist die erste Fokusebene 6 einen geringeren Abstand entlang der Mittelachse 11 relativ zu der Operationsleuchte 1 / dem Leuchtenaufnahmekörper auf als die zweite Fokusebene 9. In einer besonders vorteilhaften Ausführungsform ist der Abstand der ersten Fokusebene 6 entlang der Mittelachse 11 zur Operationsleuchte 1 / zum Leuchtenaufnahmekörper 1m und der Abstand der zweiten Fokusebene 9 entlang der Mittelachse 11 relativ zur Operationsleuchte 1 / zum Leuchtenaufnahmekörper 1,20m, besonders bevorzugt 1,40m.

In Fig. 3 sind dann zur Veranschaulichung nochmals die beiden in Fig. 1 eingeschalteten ersten Leuchten 2a und die beiden in Fig. 2 eingeschalteten zweiten Leuchten 2b gleichzeitig betätigt, so dass sich insbesondere in der ersten Fokusebene 6, die der zweiten Ausleuchtebene 17 entspricht, aber auch in der zweiten Fokusebene 9, die der ersten Ausleuchtebene 7 entspricht, ein längliches Gesamtlichtfeld 20 ergibt. Somit ist das Gesamtlichtfeld 20 in Abhängigkeit der bestromten ersten und/oder zweiten Leuchten 2a, 2b in einer bestimmten Lichtfeldanordnung / Lichtfeldgeometrie 18 ausgebildet, wobei die Lichtfeldgeometrie 18 (d.h. die Geometrie des Gesamtlichtfeldes 20) / das Gesamtlichtfeld 20 sich aus den einzelnen Teillichtfeldern 19 der einzelnen Leuchten 2a, 2b ergibt.

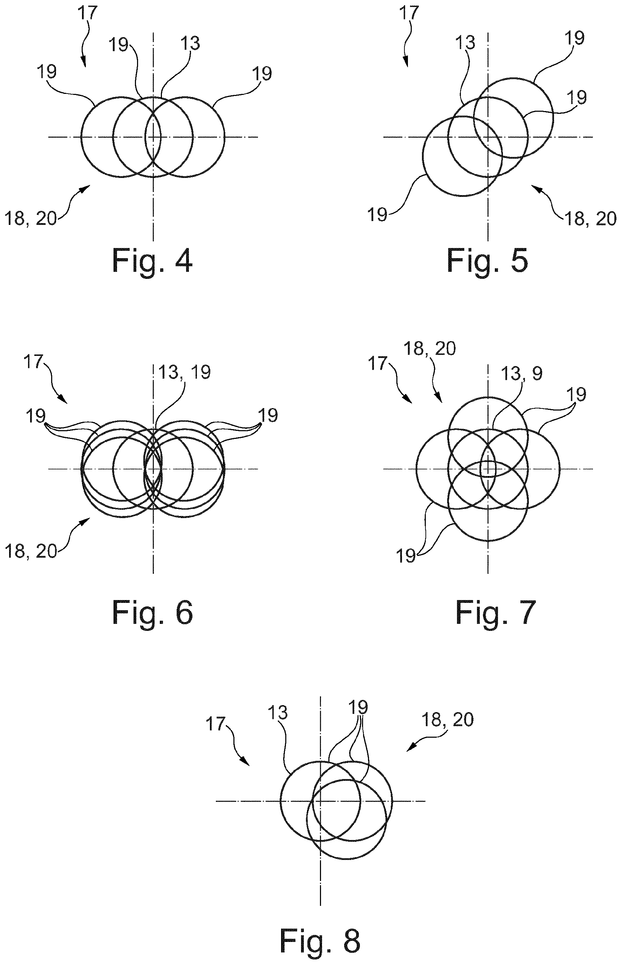

Erfindungsgemäß sind die einzelnen Leuchten 2a bzw. 2b der ersten Leuchtengruppe 5 und der zweiten Leuchtengruppe 8 innerhalb der Gruppe als auch zwischen den Gruppen unabhängig voneinander bestrombar, d.h. elektrisch ansteuerbar / betätigbar, sodass sich das erzeugte Gesamtlichtfeld 20, etwa in der jeweiligen Ausleuchtebene 7, 17 beliebig geometrisch einstellen lässt. Unter einer Einstellung der Geometrie ist hierbei sowohl eine Veränderung der Form bzw. Proportionen des Gesamtlichtfeldes 20 (d.h. der Lichtfeldgeometrie 18) zu verstehen als auch eine Veränderung der Ausrichtung des Gesamtlichtfeldes 20 (d.h. der Lichtfeldgeometrie 18) innerhalb der Ausleuchtebene 7, 17. Verschiedene mögliche Lichtfeldgeometrien 18 sind in den Fign. 4 bis 8 dargestellt.

Zur Ansteuerung der einzelnen Leuchten 2 ist in der Operationsleuchte 1, nämlich innerhalb des Leuchtenaufnahmekörpers, eine hier der Übersichtlichkeit halber nicht weiter dargestellte, zentrale Steuereinheit vorhanden, die elektrisch mit den (ersten) Leuchten 2a der ersten Leuchtengruppe 5 und elektrisch mit den (zweiten) Leuchten 2b der zweiten Leuchtengruppe 8 verbunden ist. Die Steuereinheit ist dabei vorzugsweise auch mittels mehrerer Helligkeitssensoren ausgestattet, die bspw. in dem Leuchtenaufnahmekörper oder in der Handgriffvorrichtung der Operationsleuchte 1 angeordnet sind. Die Helligkeitssensoren erfassen hierbei die gerade, tatsächlich umgesetzte Helligkeit in einem Wundfeld und geben somit der Steuereinheit Signale, die es erlauben auszurechnen, ob das Wundfeld eventuell zu hell oder zu dunkel beleuchtet ist. Dadurch erzeugt die Steuereinheit gegebenenfalls einen Steuerbefehl, der an die Leuchtengruppen 5, 8 übertragen wird und einzelne Leuchten 2 oder alle Leuchten dimmt / abschaltet oder erhellt / einschaltet. Auch lässt sich durch diese Helligkeitssensoren wiederum ein Rückschluss daraus gewinnen, welche Lichtfeldgeometrie 18, etwa gemäß der Fign. 4 bis 8, am Geeignetsten für das entsprechende Wundfeld ist.

Im Weiteren sei darauf hingewiesen, dass die Leuchten 2a bzw. 2b der ersten Leuchtengruppe 5 und/oder der zweiten Leuchtengruppe 8 unabhängig voneinander in ihrer Helligkeit / Beleuchtungsstärke regelbar / einstellbar sind. Auch sind die Leuchten 2a bzw. 2b der ersten Leuchtengruppe 5 und/oder der zweiten Leuchtengruppe 8 unabhängig voneinander betreibbar / einschaltbar und ausschaltbar. Während bspw. eine einzelne der ersten Leuchten 2a eine erste Helligkeit Beleuchtungsstärke aufweist, ist es möglich, dass eine zweite der ersten Leuchten 2a eine andere Helligkeit / Beleuchtungsstärke aufweist, etwa eine größere Helligkeit / Beleuchtungsstärke.

Auch können sich die Leuchten 2a bzw. 2b der einzelnen Leuchtengruppen 5 bzw. 8 untereinander oder zwischen den Gruppen in ihrer Lichtfarbe unterscheiden. Während bspw. einige der ersten Leuchten 2a ein bläuliches Teillichtfeld 19 erzeugen, erzeugen die anderen der ersten Leuchten 2a ein orangefarbenes Teillichtfeld 19.

In Figur 4 ist eine beispielhafte, erste Lichtfeldgeometrie 18 in einer Ausleuchtebene dargestellt, die der ersten Ausleuchtebene 7 entspricht. Hierbei sind die ersten Leuchten 2a und die zweiten Leuchten 2b (in einem ersten Ansteuerungszustand der Steuereinheit) derart betätigt, dass sich drei Teillichtfelder 19 ergeben, wobei zumindest das zentrale Teillichtfeld 19 gleichzeitig das zweite Fokuslichtfeld 16 (durch die zweiten Leuchten 2b ausgebildet) ist. Durch die teilweise überlappende Anordnung der Teillichtfelder 19 ergibt sich ein längliches Gesamtlichtfeld 20. Das Gesamtlichtfeld 20 bildet somit eine längliche, erste Lichtfeldgeometrie 18 aus.

In Fig. 5, in der eine zweite Lichtfeldgeometrie 18 in der ersten Ausleuchtebene 7 dargestellt ist, sind die ersten Leuchten 2a und die zweiten Leuchten 2b (in einem zweiten Ansteuerungszustand der Steuereinheit) derart betätigt / bestromt, dass sich drei Teillichtfelder 19 entlang einer gedachten Erstreckungsachse erstrecken und somit wiederum eine längliche (zweite) Lichtfeldgeometrie 18 erzeugen. Dabei sind jedoch andere erste Leuchten 2a angeschaltet als in Fig. 4, sodass die zweite Lichtfeldgeometrie 18 relativ zu der ersten Lichtfeldgeometrie 18 aus Fig. 4 verdreht, nämlich um etwa 45° gegen den Uhrzeigersinn verdreht, ausgerichtet ist.

In Fig. 6 sind neben den ersten Leuchten 2a und zweiten Leuchten 2b (in einem dritten Ansteuerungszustand der Steuereinheit) noch weitere Leuchten 2 anderer Leuchtengruppen angeschaltet, welche anderen Leuchtengruppen hier der Übersichtlichkeit nicht weiter dargestellt sind, jedoch wie die erste Leuchtengruppe funktionieren. Dadurch ist ein H-förmiges Gesamtlichtfeld 20 erzeugt, das sich aus sieben Teillichtfeldern zusammensetzt. Somit weist das Gesamtlichtfeld 20 eine H-förmige dritte Lichtfeldgeometrie 18 aus.

In Fig. 7 sind die ersten Leuchten 2a und die zweiten Leuchten 2b (in einem vierten Ansteuerungszustand der Steuereinheit) derart betätigt / bestromt, dass eine kreuzförmige, vierte Lichtfeldgeometrie 18 ausgebildet ist.

In Fig. 8 sind die ersten Leuchten 2a und die zweiten Leuchten 2b (in einem fünften Ansteuerungszustand der Steuereinheit) derart betätigt / bestromt, dass eine dreieckförmige, fünfte Lichtfeldgeometrie 18 ausgebildet ist.

Auch ist mit der Steuereinheit weiterhin eine Bedienungseinrichtung verbunden. Die Bedienungseinrichtung dient dafür, eine durch den Operateur / den Nutzer gewünschte Lichtfeldgeometrie 18 einzustellen. Die Bedienungseinrichtung ist bspw. mittels einer drahtgebundenen oder drahtlosen Datenverbindung, eventuell einer "Bluetooth"-Datenverbindung mit der Steuereinheit verbunden / datenübermittelnd verbunden.

In einer weiteren Ausführung enthält die Bedienungseinrichtung auch eine Spracherkennungseinheit, wodurch es möglich ist, dass die Lichtfeldgeometrie 19 über Spracheingabe einstellbar ist. Auch ist es möglich die Bedienungseinrichtung über eine "App" zu bedienen, die auf einem mobilen Gerät, wie einem Smartfon oder einem Tablet-PC installiert ist und eine Art Schieberegler darstellt, mittels dem die Leuchtengeometrie 18 einstellbar ist.

In anderen Worten ausgedrückt, ist die erfindungsgemäße Operationsleuchte 1 mit vielen Lichtquellen (LED's (anders: Leuchtdiode / Licht-emittierende Diode) in Leuchten 2 / Leuchtenmodul der Leuchten 2) ausgestattet. Jede Lichtquelle 2 hat eine Optik. Die Lichtquellen 2 sind LED's. Alle Lichtquellen 2 sind zur Haupt-/ Mittelachse 11 der OP-Leuchte 1 ausgerichtet. Ein Teil A der Lichtquellen (erste Leuchten 2a der ersten Leuchtengruppe 5), die im Zentrum der OP-Leuchte 1 angeordnet sind, bündeln das Licht auf einem Abstand X der OP-Leuchte 1 zum beleuchteten Feld, z.B. in 1m Entfernung (in erster Fokusebene 6). Ein zweiter Teil B der Lichtquellen (zweite Leuchten 2b der zweiten Leuchtengruppe 8), die um den ersten Teil 5 der Lichtquellen 2a angeordnet sind, bündeln das Licht in einem größeren Abstand Y, z.B. in 1,4m Entfernung (in zweiter Fokusebene 9). D.h.: Fig. 1: Licht aus Teil A -> kleines Lichtfeld in 1m Entfernung; Fig. 2: Licht aus Teil B -> kleines Lichtfeld in 1,4m Entfernung. Fig. 3: Licht aus Teil A + Licht aus Teil B erzeugt ein großes Lichtfeld in 1m Entfernung das sich das Licht aus Teil B um das Licht aus Teil A ergänzt. Ebenfalls wird hierdurch die Tiefenausleuchtung verbessert, da gleichzeitig durch die Erzeugung eines Lichtfeldes (Fokuslichtfelder 13, 16) im Abstand X und Y eine Art Fokuskaskade entsteht.

Man kann dieses Prinzip idealerweise noch erweitern um weitere Licht-Bereiche C und damit weitere Lichtfelder und Abstände Z. Das längliche / ovale Lichtfeld (Gesamtlichtfeld 20) wird erzeugt durch das Ansteuern einzelner Lichtquellen (erste Leuchten 2a) aus dem Bereich A und Lichtquellen aus dem Bereich B (zweite Leuchten 2b), die in einer Längsausrichtung liegen. Wenn jede LED der OP-Leuchte 1 einzeln angesteuert ist (Matrix-Ansteuerung), ist es möglich die Längsausrichtung zu verändern und das längliche Lichtfeld 20 im Raster der LED Anordnung zu drehen - Fig. 4 und 5. Es sind auch weitere Muster möglich gemäß Fign. 6 und 7 und auch außermittig nach Fig. 8.

### Bezugszeichen

- 1: Operationsleuchte
- 2: Leuchte
- 2a: erste Leuchte
- 2b: zweite Leuchte
- 3: Längsachse
- 3a: erste Längsachse
- 3b: zweite Längsachse
- 3c: dritte Längsachse
- 3d: vierte Längsachse
- 4: Lichtstrahlbündel
- 4a: erstes Lichtstrahlbündel
- 4b: zweites Lichtstrahlbündel
- 4c: drittes Lichtstrahlbündel
- 4d: viertes Lichtstrahlbündel
- 5: erste Leuchtengruppe
- 6: erste Fokusebene
- 7: erste Ausleuchtebene / Ausleuchtebene
- 8: zweite Leuchtengruppe
- 9: zweite Fokusebene
- 10: erste Umfangslinie
- 11: Mittelachse
- 12: erster Schnittpunkt
- 13: erstes Fokuslichtfeld
- 14: zweite Umfangslinie
- 15: zweiter Schnittpunkt
- 16: zweites Fokuslichtfeld
- 17: zweite Ausleuchtebene
- 18: Lichtfeldgeometrie / Lichtfeldanordnung
- 19: Teillichtfeld
- 20: Gesamtlichtfeld

## Patentansprüche

1. Operationsleuchte (1) mit einer Vielzahl, einer Leuchtengruppe (5, 8) zugeordneten, jeweils ein sich entlang einer Längsachse (3) erstreckendes Lichtstrahlbündel (4) erzeugenden Leuchten (2a, 2b), wobei mehrere erste Leuchten (2a) einer ersten Leuchtengruppe (5) zugeordnet sind und mehrere zweite Leuchten (2b) einer zweiten Leuchtengruppe (8) zugeordnet sind, und wobei sich die Längsachsen (3a, 3b) der Lichtstrahlbündel (4a, 4b) der ersten Leuchten (2a) in einer ersten gemeinsamen Fokusebene (6) schneiden und die Längsachsen (3c, 3d) der Lichtstrahlbündel (4c, 4d) der zweiten Leuchten (2b) in einer, beabstandet zu der ersten Fokusebene (6) angeordneten, zweiten gemeinsamen Fokusebene (9) schneiden, **dadurch gekennzeichnet, dass** eine Steuereinheit vorhanden ist, die elektrisch mit jeder einer Leuchtengruppe (5, 8) zugeordneten Leuchte (2a, 2b) elektrisch verbunden ist und die Leuchten (2a, 2b) jeder Leuchtengruppe (5, 8) unabhängig voneinander bestrombar sind, sodass eine durch die Leuchten (2a, 2b) erzeugte Lichtfeldgeometrie (18) in einer beabstandet zur Fokusebene (6, 9) angeordneten Ausleuchtebene (7, 17) einstellbar ist.

2. Operationsleuchte (1) nach Anspruch 1, **dadurch gekennzeichnet, dass** die Leuchten (2a; 2b) der ersten Leuchtengruppe (5) und/oder der zweiten Leuchtengruppe (8) in einem gemeinsamen Leuchtenaufnahmekörper angeordnet sind.

3. Operationsleuchte (1) nach Anspruch 1 oder 2, **dadurch gekennzeichnet, dass** die einer Leuchtengruppe (5, 8) zugeordneten Leuchten (2a, 2b) jeweils aus einem eine LED umfassenden Leuchtenmodul ausgebildet sind.

4. Operationsleuchte (1) nach einem der Ansprüche 1 bis 3, **dadurch gekennzeichnet, dass** den einer Leuchtengruppe (5, 8) zugeordneten Leuchten (2a, 2b) jeweils eine eigene Linse zugeordnet ist.

5. Operationsleuchte (1) nach einem der Ansprüche 1 bis 4, **dadurch gekennzeichnet, dass** die einer Leuchtengruppe (5, 8) zugeordneten Leuchten (2a, 2b) unabhängig voneinander in ihrer Beleuchtungsstärke einstellbar sind.

6. Operationsleuchte (1) nach einem der Ansprüche 1 bis 5, **dadurch gekennzeichnet, dass** sich zumindest einige der einer Leuchtengruppe (5, 8) zugeordneten Leuchten (2a, 2b) voneinander durch ihre Lichtfarbe unterscheiden.

7. Operationsleuchte (1) nach einem der Ansprüche 1 bis 6, **dadurch gekennzeichnet, dass** die einer Leuchtengruppe (5, 8) zugeordneten Leuchten (2a, 2b) ringförmig nebeneinander angeordnet sind.

8. Operationsleuchte (1) nach Anspruch 7, **dadurch gekennzeichnet, dass** die der ersten Leuchtengruppe (5) zugeordneten ersten Leuchten (2a) entlang einer ersten ringförmigen Umfangslinie (10) verteilt angeordnet sind sowie die der zweiten Leuchtengruppe (8) zugeordneten zweiten Leuchten (2b) entlang einer zweiten ringförmigen Umfangslinie (14) verteilt angeordnet sind.

9. Operationsleuchte (1) nach Anspruch 8, **dadurch gekennzeichnet, dass** die erste Umfangslinie (10) innerhalb der zweiten Umfangslinie (14) angeordnet ist.

10. Operationsleuchte (1) nach einem der Ansprüche 1 bis 9, **dadurch gekennzeichnet, dass** eine Bedienungseinrichtung vorhanden ist, mittels der die Lichtfeldgeometrie (18) einstellbar ist.

11. Operationsleuchte (1) nach Anspruch 10, **dadurch gekennzeichnet, dass** die Bedienungseinrichtung eine Spracherkennungseinheit aufweist.

12. Operationsleuchte (1) nach Anspruch 11, **dadurch gekennzeichnet, dass** die Bedienungseinrichtung mittels einer drahtgebundenen oder drahtlosen Datenverbindung mit der Steuereinheit verbunden ist.

## Claims

1. A surgical light (1) comprising a plurality of lights (2a, 2b) associated with a light group (5, 8), said lights (2a, 2b) each producing a light beam (4) extending along a longitudinal axis (3), wherein several first lights (2a) are associated with a first light group (5) and several second lights (2b) are associated with a second light group (5), and wherein the longitudinal axes (3a, 3b) of the light beams (4a, 4b) of the first lights (2a) intersect in a first common focal plane (6) and the longitudinal axes (3c, 3d) of the light beams (4c, 4d) of the second lights (2b) intersect in a second common focal plane (9) arranged at a distance from the first focal plane (6), **characterized in that** a control unit is provided which is electrically connected to each light (2a, 2b) associated with a light group (5, 8) and the lights (2a, 2b) of each light group (5, 8) can be supplied with current independently of each other such that a light field geometry (18) produced by the lights (2a, 2b) in an illumination plane (7, 17) arranged at a distance from the focal plane (6, 9) can be adjusted.

2. The surgical light (1) according to claim 1, **characterized in that** the lights (2a; 2b) of the first light group (5) and/or of the second light group (8) are arranged in a common light receiving member.

3. The surgical light (1) according to claim 1 or 2, **characterized in that** each of the lights (2a, 2b) associated with a light group (5, 8) is formed of a light module comprising an LED.

4. The surgical light (1) according to one of the claims 1 to 3, **characterized in that** a respective separate lens is associated with each of the lights (2a, 2b) associated with a light group (5, 8).

5. The surgical light (1) according to one of the claims 1 to 4, **characterized in that** the lights (2a, 2b) associated with a light group (5, 8) can be adjusted independently of each other with regard to their illuminance.

6. The surgical light (1) according to one of the claims 1 to 5, **characterized in that** at least several of the lights (2a, 2b) associated with the one light group (5, 8) are different from each other in their luminous color.

7. The surgical light (1) according to one of the claims 1 to 6, **characterized in that** the lights (2a, 2b) associated with one light group (5, 8) are arranged next to each other in ring shape.

8. The surgical light (1) according to claim 7, **characterized in that** the first lights (2a) associated with the first light group (5) are arranged to be spread along a first ring-shaped peripheral line (10) and the second lights (2b) associated with the second light group (8) are arranged to be spread along a second ring-shaped peripheral line (14).

9. The surgical light (1) according to claim 8, **characterized in that** the first peripheral line (10) is arranged inside the second peripheral line (14).

10. The surgical light (1) according to one of the claims 1 to 9, **characterized in that** an operating unit is provided by means of which the light field geometry (18) can be adjusted.

11. The surgical light (1) according to claim 10, **characterized in that** the operating unit includes a speech recognition unit.

12. The surgical light (1) according to claim 11, **characterized in that** the operating unit is connected to the control unit by means of a wired or wireless data communication.

## Revendications

1. Scialytique (1) pourvu d'une pluralité de lampes (2a, 2b) associées à un groupe de lampes (5, 8), lesquelles produisant chacune un faisceau de rayons lumineux (4) qui s'étend le long d'un axe longitudinal (3), dans lequel plusieurs premières lampes (2a) sont associées à un premier groupe de lampes (5) et plusieurs secondes lampes (2b) sont associées à un second groupe de lampes (8), et dans lequel les axes longitudinaux (3a, 3b) des faisceaux de rayons lumineux (4a, 4b) des premières lampes (2a) se coupent dans un premier plan de focalisation commun (6) et les axes longitudinaux (3c, 3d) des faisceaux de rayons lumineux (4c, 4d) des secondes lampes (2b) se coupent dans un second plan de focalisation commun (9) agencé à distance du premier plan de focalisation (6), **caractérisé en ce qu'**il existe une unité de commande qui est raccordée électriquement à chaque lampe (2a, 2b) associée à un groupe de lampes (5, 8) et les lampes (2a, 2b) de chaque groupe de lampes (5, 8) peuvent être alimentées en courant indépendamment les unes des autres si bien qu'une géométrie de champ lumineux (18) produite par les lampes (2a, 2b) peut être ajustée dans un plan d'éclairage (7, 17) agencé à distance du plan de focalisation (6, 9).

2. Scialytique (1) selon la revendication 1, **caractérisé en ce que** les lampes (2a, 2b) du premier groupe de lampes (5) et/ou du second groupe de lampes (8) sont agencées dans un corps de logement de lampes commun.

3. Scialytique (1) selon les revendications 1 ou 2, **caractérisé en ce que** les lampes (2a, 2b) associées à un groupe de lampes (5, 8) sont réalisées respectivement comme un module de lampes comprenant une LED.

4. Scialytique (1) selon l'une quelconque des revendications 1 à 3, **caractérisé en ce qu'**une lentille propre est attribuée respectivement aux lampes (2a, 2b) associées à un groupe de lampes (5, 8).

5. Scialytique (1) selon l'une quelconque des revendications 1 à 4, **caractérisé en ce que** les lampes (2a, 2b) associées à un groupe de lampes (5, 8) sont réglables indépendamment les unes des autres au niveau de leur intensité d'éclairage.

6. Scialytique (1) selon l'une quelconque des revendications 1 à 5, **caractérisé en ce qu'au** moins quelques-unes des lampes (2a, 2b) associées à un groupe de lampes (5, 8) se différencient les unes des autres par leur couleur lumineuse.

7. Scialytique (1) selon l'une quelconque des revendications 1 à 6, **caractérisé en ce que** les lampes (2a, 2b) associées à un groupe de lampes (5, 8) sont disposées de manière annulaire les unes à côté des autres.

8. Scialytique (1) selon la revendication 7, **caractérisé en ce que** les premières lampes (2a) associées au premier groupe de lampes (5) sont disposées en étant réparties le long d'une première ligne périphérique annulaire (10), de même que les secondes lampes (2b) associées au second groupe de lampes (8) sont disposées en étant réparties le long d'une seconde ligne périphérique annulaire (14).

9. Scialytique (1) selon la revendication 8, **caractérisé en ce que** la première ligne périphérique (10) est agencée à l'intérieur de la seconde ligne périphérique (14).

10. Scialytique (1) selon l'une quelconque des revendications 1 à 9, **caractérisé en ce qu'il** existe un dispositif d'actionnement qui permet de régler la géométrie de champ lumineux (18).

11. Scialytique (1) selon la revendication 10, **caractérisé en ce que** le dispositif d'actionnement présente une unité de reconnaissance vocale.

12. Scialytique (1) selon la revendication 11, **caractérisé en ce que** le dispositif d'actionnement est raccordé à l'unité de commande au moyen d'une liaison de données filaire ou sans fil.
